(19) Europäisches Patentamt

European Patent Office

Office européen des brevets

(11) **EP 3 959 510 B1**

(12) **EUROPEAN PATENT SPECIFICATION**

(45) Date of publication and mention
of the grant of the patent:
**25.02.2026 Bulletin 2026/09**

(21) Application number: **20723480.8**

(22) Date of filing: **22.04.2020**

(51) International Patent Classification (IPC):
***G01N 22/00*** *(2006.01)*

(52) Cooperative Patent Classification (CPC):
**G01N 22/00**

(86) International application number:
**PCT/GB2020/050994**

(87) International publication number:
**WO 2020/217050 (29.10.2020 Gazette 2020/44)**

(54) **MICROWAVE-BASED METHOD AND APPARATUS FOR MONITORING A PROCESS VARIABLE**

MIKROWELLENBASIERTES VERFAHREN UND VORRICHTUNG ZUR ÜBERWACHUNG EINER PROZESSVARIABLEN

PROCÉDÉ ET APPAREIL À BASE DE MICRO-ONDES POUR SURVEILLER UNE VARIABLE DE PROCESSUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.04.2019 GB 201905770**

(43) Date of publication of application:
**02.03.2022 Bulletin 2022/09**

(73) Proprietor: **Rosemount Tank Radar AB**
**435 23 Mölnlycke (SE)**

(72) Inventor: **ZHANG, Jingdong**
**Slough Berkshire SL3 8WY (GB)**

(74) Representative: **Kransell & Wennborg KB**
**P.O. Box 2096**
**403 12 Göteborg (SE)**

(56) References cited:
**EP-A1- 1 083 414       GB-A- 2 179 454**
**GB-A- 2 527 794        US-A- 5 675 259**
**US-A1- 2015 276 460    US-B2- 9 556 731**

• **YANSHENG XU ET AL: "ON THE MEASUREMENT OF MICROWAVE PERMITTIVITY OF BIOLOGICAL SAMPLES USING NEEDLE-TYPE COAXIAL PROBES", IEEE TRANSACTIONS ON INSTRUMENTATION AND MEASUREMENT, IEEE SERVICE CENTER, PISCATAWAY, NJ, US, vol. 42, no. 4, August 1993 (1993-08-01), pages 822 - 827, XP000399898, ISSN: 0018-9456, DOI: 10.1109/ 19.234492**

**Description**

*Field of the Invention*

This invention relates to a microwave-based sensor for monitoring a process variable. By way of example only, embodiments of the invention may serve to monitor moisture content, sugar content and salt concentration

*Background to the Invention*

There is a well-known and widespread requirement for devices that can detect changes in parameters of a medium, such parameters also known as a process variables. In the context of this document the term process variable is intended to mean any variation in a physical parameter of a medium due to external effects and/or age. Particular process variables to which the invention has particular application include, but are not necessarily limited to, bulk density, moisture content, liquid concentration, sugar concentration, salt concentration and oil quality.

Current sensor technology includes infrared ray, ultrasonics, magnetic induction, Hall effect sensors and radio frequency-based devices. These technologies all have their place but many industrial processes require online monitoring of material parameters under high process temperatures and pressures. Under these conditions exiting technologies exhibit reliability problems.

Variations in complex permittivity of solid particles or liquids, using a radio-frequency open-ended coaxial sensor, is a popular means used to detect changes in material composition. US Patent 5,675,259 discloses a microwave probe based on a radio-frequency open-ended coaxial waveguide for monitoring the presence, concentration and flow in a flowline or in a holding container. The microwave sensor is designed for operating in a wide frequency band from zero to the upper limited frequency (*f*) using chosen waveguide diameters and selected insulation of particular permittivity, however the difference in reflected amplitude and phase is very small when the frequency is less than 1GHz, frequency being determined by the expression:

$$f = c/(\pi * \sqrt{\varepsilon} * (a + b))$$

where c is the speed of light in a vacuum; $\varepsilon$ is the permittivity of the insulation material; a is the diameter of the centre conductor; and b is the inside diameter of the outer conductor. The probe in this example is designed to an impedance that is as constant as possible along the length of the probe, and in the order of 50 ohm.

US Patent 8,629,681 proposes a microwave sensor and algorithm to detect the bulk density and moisture content in granular or particulate material, especially agricultural commodities.

With existing forms of open-ended coaxial waveguide, the difference in reflected amplitudes from media with different complex permittivities is only about 4 - 6dB. As a result, high quality and costly electronic processing is required to distinguish reflected amplitudes and phase changes due to changes in media. When the permittivity of the medium is less than 5, it is challenging to implement an electronics circuitry and algorithm to effect reliable on-site differentiation.

A further factor requiring consideration is that traditional forms of open-ended coaxial sensors rely for their use on algorithms that derive material permittivity from the amplitude and phase of the return loss. The resulting permittivity is then used to monitor the change in material property. These algorithms require pre-calibration of the sensor using a number of different materials, each having known permittivity, so that some calibration constants can be determined. However, in subsequent use of the sensor, these constants are known sometimes to change with temperature and other variations in site condition, leading to errors in process control.

It is an object of the invention to provide a method and/or apparatus to check switch function that will go at least some way in addressing the aforementioned problems; or which will at least provide a novel and useful choice.

US 2015/276460 A1 relates to a coaxial feed-through device for coupling a received process connection to a storage tank including an inner electrical conductor, an outer electrical conductor; and a dielectric sleeve disposed between the probe and the outer electrical conductor. The dielectric sleeve is configured to provide an upper coaxial transmission line segment providing a substantially 50 ohm impedance and a lower coaxial transmission line segment which includes one or more sub-segments having an impedance that is at least forty percent higher as compared to the substantially 50 ohm impedance.

GB 2 527 794 A relates to a system and method that permits measuring properties and thickness of a dielectric layer, particularly a dielectric layer close to a pipeline wall, and more particularly fluids flowing inside a pipe is provided. The system comprises a sensor operating in a material characterization mode in a first frequency range and a sensor operating in a thickness characterization mode in a second frequency range.

EP 1 083 414 A1 relates to a level measuring instrument for measuring a level of a filled material in a container, in the case of which electromagnetic signals generated by means of an electronic circuit are guided into the container via a waveguide and signals reflected at a filled material surface are guided out, in the case of which an impedance-matching device active in a frequency band of a bandwidth is connected upstream of the waveguide. For this purpose, the level measuring instrument comprises an injector, which transmits the signals from the circuit to the waveguide, and which has an inner conductor, which inner conductor has, for the purpose of increasing the characteristic impedance, a conductor section with a geometry deviating from a cylindrical shape.

Summary of the Invention

Accordingly, in a first aspect, the invention provides apparatus defined by claim 1.

Preferably said sensor includes a main waveguide section of impedance Z and wherein said impedance mismatch is at least 1.5Z and preferably substantially 4Z.

Preferably said impedance mismatch is effected by a resonant cavity located between said main waveguide section and said connection end.

Preferably said resonant cavity is evacuated, or filled with air or a low-permittivity foamed material.

Preferably said impedance mismatch has a dimension in the direction of length L of a multiple of substantially $1/4\lambda$.

Preferably said main waveguide section has a dimension in the direction of L which is a multiple of substantially $1/4 \lambda$.

Preferably said apparatus further includes an expansion section formed at said distal end in which said inner electrode is expanded in diameter and said outer electrode is reduced in thickness to accommodate the expansion.

Preferably the dimension of said expansion section in the direction of L is a multiple of substantially $1/4 \lambda$.

Preferably an insulating coating is provided over said distal end to prevent short-circuiting between said inner and outer conductors.

Preferably the electrical insulation between said outer and inner electrodes comprises a thermal insulating material.

Preferably said sensor further includes a thermal insulation section at the connection end thereof.

Preferably said processing electronics is configured to initiate an output derived solely from amplitudes of return loss.

Alternatively said processing electronics is configured to initiate an output derived from amplitude and phase changes of return loss.

Preferably said processing electronics is configured to apply a peak fitting algorithm to peaks of return loss, to then determine changes of amplitude and/or bandwidth at those peaks of return loss, and thereby provide an indication of change in process variable.

In a second aspect the invention provides apparatus operable to monitor a change in process variable in a medium, said apparatus including a microwave sensor configured and arranged to interact with the medium, and drive and processing electronics configured and operable to provide drive signals to said sensor, to receive and process received signals from said sensor, and to initiate an output, wherein said sensor comprises an open ended microwave waveguide formed by an outer electrode and an inner electrode held co-axially within said outer electrode with electrical insulation there-between, said sensor having a connection end for connection to said processing electronics, a distal end for mounting in substantial contact with a wall of a non-conductive vessel containing said medium or in substantial contact with a non-conductive window in a wall of a vessel containing said medium, said sensor having a length L and wherein said sensor is configured to operate within a frequency band having a frequency $f_0$ with wavelength $\lambda$ located substantially centrally within said band, said sensor including at least one impedance mismatch having a dimension in the direction of length L related to $\lambda$ and wherein said process electronics is configured to initiate an output derived from amplitudes of return loss.

Preferably said apparatus includes one or more features as set forth above.

Many variations in the way the present invention can be performed will present themselves to those skilled in the art. The description which follows is intended as an illustration only of one means of performing the invention and Subject to the scope of the appended claims, the lack of description of variants or equivalents should not be regarded as limiting.

*Brief Description of the Drawings*

One working embodiment of the invention will now be described with reference to the accompanying drawings in which:

Figure 1:    shows a cross-section through a prior art open-ended waveguide sensor;

Figure 2:    shows a cross-section through an open-ended waveguide sensor according to the invention;

Figure 3: shows simulated comparisons of amplitudes of return losses of a sensor according to the invention, and a prior art sensor, respectively operating in media of differing permittivities;

Figure 4: shows plots of return losses derived from samples of basmati rice of differing moisture contents;

Figure 5: shows plots of resonant frequency change derived by applying curve fitting to the data shown in Figure 4;

Figure 6: shows plots of bandwidth change derived by applying curve fitting to the data shown in Figure 4;

Figure 7: shows an example of drive and processing circuit which may be used to implement the invention; and

Figure 8: shows a curve fitting technique used in the derivation of Figures 5 & 6

*Detailed Description of Working Embodiment*

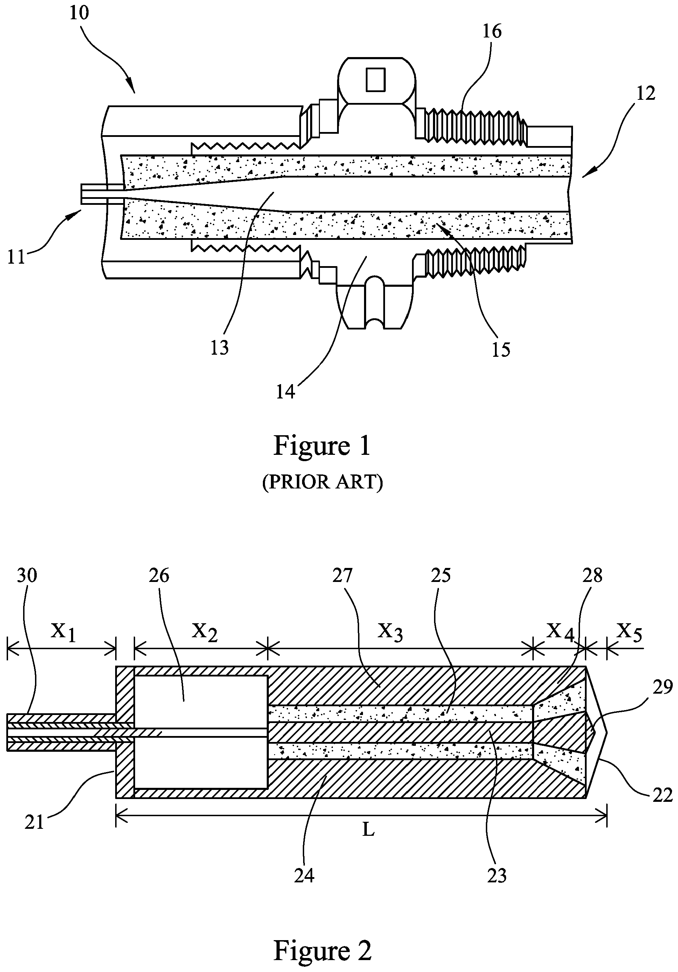

Referring firstly to Fig 1, a typical prior art open-ended waveguide sensor 10 is shown in Figure 1 but reference can also be made to US Patent 5,675,259 which contains a detailed description of the construction and operation of such a sensor as well as various industrial applications in which a sensor of this type can be used. In the form shown in Fig 1, the sensor 10 has a connection end 11 and a distal end 12 for contact with a fluid which, in the context of this disclosure, includes gases, liquids and fine solids such as powders. The sensor 10 includes an inner electrode 13, an outer electrode 14, the electrodes 13 and 14 held in spaced relationship (typically co-axial relationship) by an electrically insulating material 15. The electrodes are typically formed from a $_{20}$ metal such as stainless steel and the insulating material 15 may comprise a suitable plastics material such as polytetrafluoroethylene (PTFE), or a suitable ceramic.

The outer electrode 14 may comprise the outer body of the sensor which, in the form shown, includes a threaded section 16 to allow the sensor to be mounted in the position in which it is to be used. Typically this will be an aperture of a relatively restricted diameter in the wall of a process vessel.

The sensor shown in Fig 1 is configured according to design rules which dictate that the impedance of the sensor should be kept as constant as possible along its length and that the sensor should operate over a wide frequency band with the highest possible cut-off frequency; that is to say the highest frequency to maintain transverse electromagnetic (TEM) mode. As will become apparent from the description that follows, this limits the usefulness of such a sensor forming part of an instrument for monitoring process variables.

Turning now to Fig 2, an open-ended wave-guide sensor 20 incorporated in a level switch according to the invention is configured to different design rules, namely:

i) The sensor should be optimized around a central working frequency $f_0$ with wavelength $\lambda$;

ii) the sensor should include a high impedance section having a dimension along the length of the sensor that is a multiple of ¼ $\lambda$;

iii) the lengths of the various sections of the sensor should be multiples of $^1/_4\ \lambda$;

iv) the ratio of diameters of the inner and outer electrodes should be chosen to provide a cut-off frequency that is as high as possible above $f_0$; and

v) the lateral dimension of the sensor at the distal end should be expanded to improve the sensitivity of the sensor to media of low permittivity.

The sensor 20 has a connection end 21 for connection to drive and processing electronics, a distal end 22 for contact with the media, an inner electrode 23 and an outer electrode 24, the electrodes 23 and 24 being separated by an insulator 25. As with the prior art sensor the electrodes may be formed from stainless steel and the insulator 25 from PTFE, ceramic or other suitable material. For higher temperature applications it is preferred to form the electrodes from titanium, in which case the insulation should be of material having a very similar thermal expansion coefficient. An example of such a material is Ceramit® 14, a material available from Ceramic Substrates and Components Limited of Newport, Isle of Wight, UK.

It should be emphasised that other materials may be used and that the scope of the invention is not to be limited to particular combinations of electrode material and insulation composition.

The sensor 20 is comprised of a number of different sections arranged along length L and configured to significantly improve performance over the prior art sensors mentioned. At or adjacent to connection end 20 is a reflection section in the form of resonant cavity 26 that provides an impedance mismatch. The sensor further includes a main waveguide section 27, and a tip $_{15}$ section 28. As can be seen, the tip section 28 is preferably domed in shape by projecting the inner electrode 23 out beyond the outer electrode 24 at 29 by an amount of around $^1/_{10}\lambda$ or less, and forming the insulation between the electrodes to encourage liquids to drop off the distal end 22 of the sensor, the tip section preferably being provided with a thin coating of a suitable polymer 20 such as, for example, polyfluorenylene ethynylene (PFE), or in high temperature applications, a thin layer of Ceramit 14.

For applications in which high temperatures are anticipated, the connection end may be provided with a thermal insulation section 30 formed with a glassmetal seal to reduce bring down the temperature from the working parts of the 25 sensor before the sensor is connected to the drive and processing facility.

The reflection section or resonant cavity 26 is configured to generate a resonant standing wave around the frequency $f_0$ in the main sensor body through a significant impedance change or mismatch, the impedance of this section being at least $1^1/_2$ times that of the main waveguide and more preferably 3 to 4 times the impedance of the main waveguide section. The impedance mismatch may be achieved by evacuating the cavity or filling it with air or a low permittivity material such as foam, examples of which include a low dielectric open cell foam such as, for example, Cuming Microwave C-Stock or Eccostock FFP. The length dimension of the section 26 is preferably a multiple of ¼λ.

As indicated in Fig 2, the thermal insulation section 30, reflection section 26, main waveguide section 27, tip section 28, and domed end 29 have dimensions $X_1, X_2, X_3, X_4$ and $X_5$ respectively in the direction of L. Performance of the sensor is enhanced if $X_2$ is a multiple of ¼λ while $X_3$, and $X_4$ are multiples of ½λ. $X_5$ is preferably in the order of $^1/_{10}\,\lambda$.

In order to achieve a distinguishable difference in reflected amplitude and preferably phase induced by a permittivity change of the process media, especially media with a permittivity in the range of about 2 - 3, the performance of the proposed sensor has been optimised in a narrow frequency band to its centre frequency $f_0$, say 4.5GHz, or to its resonant frequency of 3.2GHz or 5.7GHz.

The resonant cavity 26 has more than at least 1.5 times the impedance of the main co-axial waveguide section and preferably in the order of four times the impedance. The resonant cavity is conveniently formed by altering the conductor or electrode dimensions and changing the insulating properties. The length of the resonant cavity is preferably a multiple of a quarter of the wavelength of the centre working frequency.

By way of example only, a sensor configured for mounting through an industry-standard 1'' diameter mounting hole may have an effective 24 mm outer diameter, a maximum internal diameter of 20 mm, titanium electrodes and Ceramit 14 insulation and may be configured such that the various sections have the following dimensions:

| Section | a (mm) | b (mm) | ε | f (GHz) | Z (ohm) |
|---|---|---|---|---|---|
| Thermal Insulation | 7 | 1 | 5.2 | 10.5 | 51.2 |
| Reflection Section | 22 | 1.5 | 1 | 8. | 161.1 |
| Main waveguide | 7 | 2 | 5.2 | 4.9 | 31.0 |
| Expansion section | 24 | 7 | 5.2 | 2.7 | 32.4 |

Where a is the outside diameter of the insulation, b is the outside diameter of 10 the inner conductor, ε is permittivity, *f* is cut-off frequency and Z is impedance.

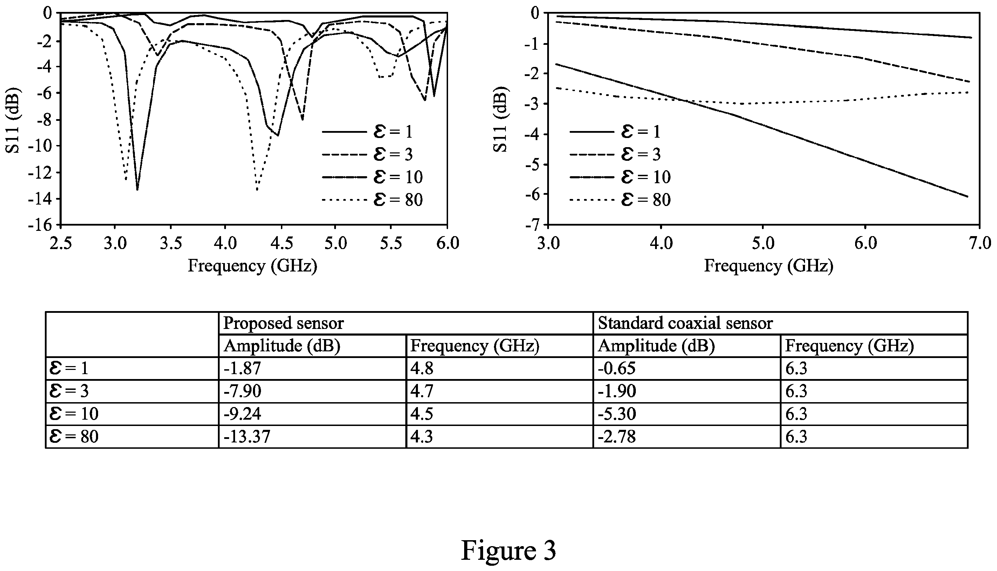

A simulated comparison of the performance of the sensor proposed herein, with a prior art sensor of the type described in US patent 5,675,259 is shown in Figure 3 and it can be seen that the S11 return losses at different permittivities using the proposed sensor occur at clearly defined frequencies when compared with the same return losses measured with the prior art sensor. As is well known by those skilled in the art, when a transverse electromagnetic wave (TEM) is transmitted along the sensor body and reflected at the distal end 22, the amplitude and phase of the reflected wave will vary depending on the permittivity of the medium surrounding the distal end 22. As described above prior art instruments used in monitoring process variables have first determined the change in permittivity and, from that, determined the change in process variable. The present invention provides a combination of novel sensor and algorithm that allows differences 25 in variable to be established directly from the amplitudes of return losses while at the same time allowing the use of phase information to be included in the calculation, if desired.

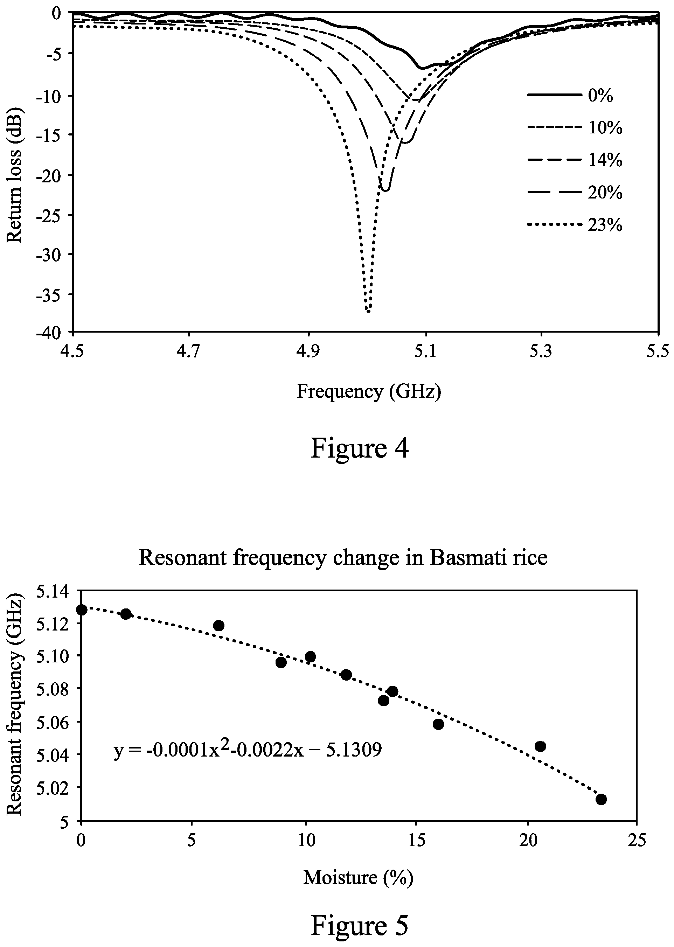

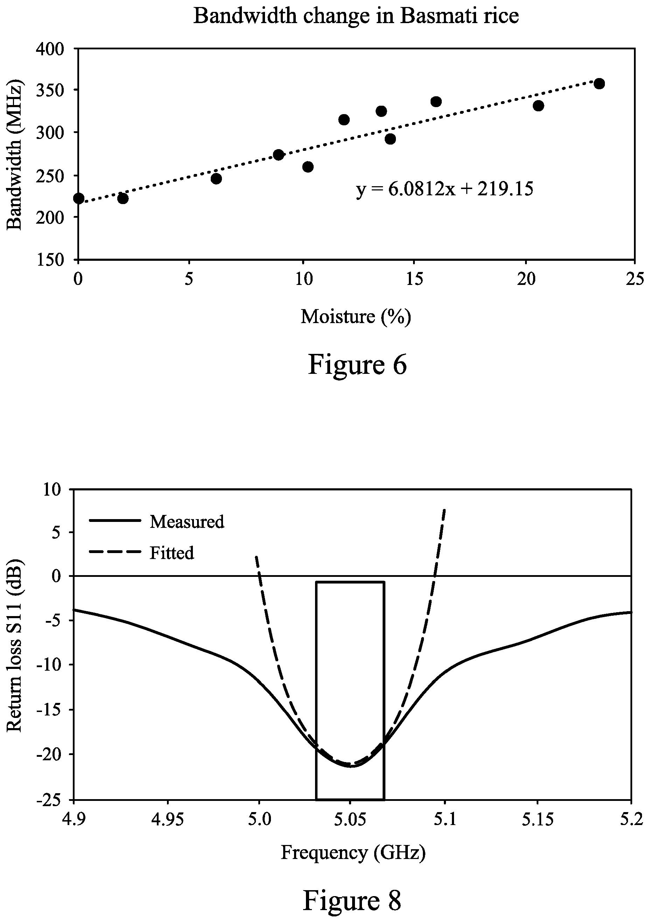

Referring now to Figures 4 to 6, an example is shown of the invention being applied to the determination of moisture content in basmati rice. Figure 4 shows plots of amplitudes of return loss against frequency, which are established before installation of the sensor by direct measurements taken using samples of basmati rice having different levels of moisture content. It can be seen that for each moisture level the amplitude of the return loss reaches a clearly defined minimum peak at a clearly defined frequency. By applying a typical Gaussian peak fitting to each negative peak, two relationships can be established, the first being a 2nd order polynomial plot of resonant frequency against moisture content as shown in Figure 5, and the second being a linear plot of bandwidth against moisture content as shown in Figure 6. Once the relationship of resonant frequency or bandwidth has been established by the polynomial fitting formulae, any moisture content between 0% and 23% can be derived by direct measurement of resonant frequency and/or bandwidth.

To improve the accuracy of moisture measurement with change in resonant frequency, the curve-fitting algorithm is designed to draw peak and bandwidth information from a number of measurement points around the peak resonant frequency. An example of the procedure adopted is:

i) Data points are chosen around the resonant frequency peak to a level around 4dB above the dip point of S11 as shown in the block in Figure 8;

ii) The curve fitting algorithm Gaussian peak fitting algorithm is then applied to find coefficients a1, b1 & c1 in the equation:

$$a1 * \exp\left(-\frac{(x-b1)^2}{c1^2}\right)$$

iii) Calculate the peak frequency from the fitted curve, $f_{peak} = b1$;

iv) Calculate the -3dB full bandwidth from the fitted curve, BW = 1.665* c1

Either peak or bandwidth will vary with changes in material parameter e.g. moisture content as permittivity changes. These changes can be used to characterize the parameter of the material, in this case moisture content, on site from the plots shown in Figures 5 and 6.

Note that by only using amplitude information, a full calibration over a range of permittivities for each material is not required and the system constants are not sensitive to the conditions in which the sensor is operating. Shifts in resonant frequency are proportional to changes in the real part of permittivity whereas the changes in bandwidth are proportional to the imaginary part of permittivity.

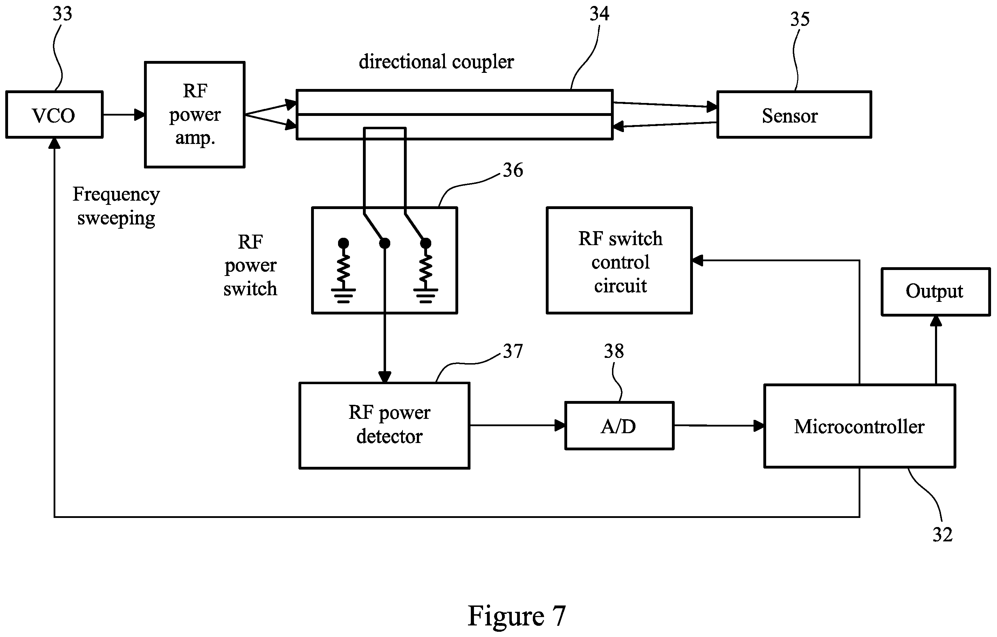

This also leads to a simplified radio frequency (RF) drive and processing circuit in which the need for an RF frequency mixer and I/Q modulation means can be eliminated if phase information is not required for the determination of material characteristic. An example of circuit which can be used to implement the invention is shown in Figure 7.

As shown in Figure 7, microcontroller 32 sends a signal to voltage-controlled oscillator 33 to start frequency sweeping in pre-determined frequency steps within a frequency band. The resulting RF signals are directed to directional coupler 34 in which they are split into two parts in a known ratio, one part going directly to sensor 35 to be reflected back when it meets the distal end of the sensor in contact with, or adjacent to, the medium under observation. The other part of the RF signal is directed to RF power switch 36 as a reference signal to the sensor. Through operation of switch 36 the reference and reflected signals are directed in turn to RF power detector 37, analogue to digital convertor 38 and then to the microcontroller 32. The microcontroller 32 then calculates the power ratio between reflected and reference signals as the amplitude of return loss S11 for each frequency step.

It thus follows that the circuit described is a calibration-free return loss amplitude measurement circuit.

Whilst the above description may be interpreted as applying to apparatus mounted through an aperture in a process vessel, the invention may also be applied to apparatus mounted in a non-penetrating manner on the outer surface of a vessel which is formed from a non-conducting material such as, for example, plastic, glass or ceramic; or mounted on a non-conducting window included in the wall of a vessel otherwise formed from a conducting material. In such situations a lower design frequency $f_0$, say around 3.5GHz is preferred as it will allow the microwave energy to penetrate the container wall and generate a large effective sensing volume more easily.

## Claims

1. Apparatus operable to monitor a change in process variable in a medium in contact with said apparatus, said apparatus including:

    a microwave sensor having a connection end (21) and a distal end (22) for contact with the medium; and drive and processing electronics connected to the connection end (21) of the microwave sensor and configured and operable to provide drive signals to the connection end (21) of said sensor, to receive and process received signals resulting from reflection of the drive signals at the distal end (22) of said sensor, and to initiate an output, wherein said sensor comprises an open ended microwave waveguide formed by an outer electrode (24) and an inner electrode (23) held co-axially within said outer electrode (24) with electrical insulation (25) there-between, said sensor having a length L,

wherein said sensor is configured to operate within a frequency band having a frequency f0 with wavelength $\lambda$ located substantially centrally within said band, said sensor including at least one impedance mismatch having a dimension in the direction of length L related to $\lambda$,

**characterized by** that

at said distal end (22), said inner electrode (23) projects beyond said outer electrode (24) in the direction of L by an amount of around $1/10\lambda$ or less, and

wherein said process electronics is configured to initiate an output derived from amplitudes of return loss of the received signals in relation to the drive signals.

2. Apparatus as claimed in claim 1 wherein said sensor includes a main waveguide section (27) of impedance Z and wherein said impedance mismatch is least 1.5Z and preferably substantially 4Z.

3. Apparatus as claimed in claim 2 wherein said impedance mismatch is effected by a resonant cavity (26) located between said main waveguide (27) section and said connection end (21).

4. Apparatus as claimed in claim 2 or claim 3 wherein said resonant cavity (26) is evacuated, or filled with air or a low-permittivity foamed material.

5. Apparatus as claimed in any one of claims 1 to 4 wherein said impedance mismatch has a dimension in the direction of length L of a multiple of substantially $1/4\lambda$.

6. Apparatus as claimed in any one of claims 2 to 5 wherein said main waveguide section (27) has a dimension in the direction of L which is a multiple of substantially $1/4\lambda$.

7. Apparatus as claimed in any one of the preceding claims further including an expansion section (28) formed at said distal end (22) in which said inner electrode (23) is expanded in diameter and said outer electrode (24) is reduced in thickness to accommodate the expansion.

8. Apparatus as claimed in claim 7 wherein the dimension of said expansion section (28) in the direction of L is a multiple of substantially $1/4\lambda$.

9. Apparatus as claimed in any one of the preceding claims wherein an insulating coating is provided over said distal end (28) to prevent short-circuiting between said inner (23) and outer (24) conductors.

10. Apparatus as claimed in any one of the preceding claims wherein the electrical insulation (25) between said outer (24) and inner (23) electrodes comprises a thermal insulating material.

11. Apparatus as claimed in any one of the preceding claims wherein said sensor further includes a thermal insulation section (30) at the connection end (21) thereof.

12. Apparatus as claimed in any one of the preceding claims wherein said processing electronics is configured to initiate an output derived solely from amplitudes of return loss.

13. Apparatus as claimed in any one of claims 1 to 11 wherein said processing electronics is configured to initiate an output derived from amplitude and phase changes of return loss.

14. Apparatus as claimed in any one of the preceding claims wherein said processing electronics is configured to apply a peak fitting algorithm to peaks of return loss, to then determine changes of amplitude and/or bandwidth at those peaks of return loss, and thereby provide an indication of change in process variable.

**Patentansprüche**

1. Vorrichtung, die zum Überwachen einer Änderung einer Prozessvariablen in einem Medium, das mit der Vorrichtung in Kontakt steht, betreibbar ist, wobei die Vorrichtung aufweist:

   einen Mikrowellensensor mit einem Verbindungsende (21) und einem distalen Ende (22) zum Kontakt mit dem Medium; und

Ansteuerungs- und Verarbeitungselektronik, die mit dem Verbindungsende (21) des Mikrowellensensors verbunden ist und eingerichtet und betreibbar ist, um Ansteuerungssignale an das Verbindungsende (21) des Sensors zu liefern, um empfangene Signale zu empfangen und zu verarbeiten, die sich aus einer Reflexion der Ansteuerungssignale an dem distalen Ende (22) des Sensors ergeben, und um eine Ausgabe auszulösen, wobei der Sensor einen offenendigen Mikrowellenleiter umfasst, der durch eine äußere Elektrode (24) und eine innere Elektrode (23), die koaxial innerhalb der äußeren Elektrode (24) gehalten ist, mit einer elektrischen Isolierung (25) dazwischen gebildet ist, wobei der Sensor eine Länge L aufweist, wobei der Sensor eingerichtet ist, um innerhalb eines Frequenzbandes mit einer Frequenz f0 zu arbeiten, wobei eine Wellenlänge λ im Wesentlichen in der Mitte des Bandes liegt, wobei der Sensor mindestens eine Impedanzfehlanpassung mit einer Abmessung in der Richtung der Länge L in Bezug auf λ aufweist, **dadurch gekennzeichnet, dass** an dem distalen Ende (22) die innere Elektrode (23) über die äußere Elektrode (24) in der Richtung von L um einen Betrag von etwa 1/10λ oder weniger vorsteht, und wobei die Verarbeitungselektronik eingerichtet ist, um eine Ausgabe auszulösen, die aus Amplituden einer Rückflussdämpfung der empfangenen Signale in Bezug auf die Ansteuerungssignale abgeleitet wird.

2. Vorrichtung nach Anspruch 1, wobei der Sensor einen Hauptwellenleiterabschnitt (27) mit einer Impedanz Z aufweist und wobei die Impedanzfehlanpassung mindestens 1,5 Z und vorzugsweise im Wesentlichen 4 Z beträgt.

3. Vorrichtung nach Anspruch 2, wobei die Impedanzfehlanpassung durch einen Resonanzhohlraum (26) bewirkt wird, der sich zwischen dem Hauptwellenleiterabschnitt (27) und dem Verbindungsende (21) befindet.

4. Vorrichtung nach Anspruch 2 oder Anspruch 3, wobei der Resonanzhohlraum (26) evakuiert oder mit Luft oder einem geschäumten Material mit niedriger Dielektrizitätskonstante gefüllt ist.

5. Vorrichtung nach einem der Ansprüche 1 bis 4, wobei die Impedanzfehlanpassung eine Abmessung in der Richtung der Länge L aufweist, die ein Vielfaches von im Wesentlichen 1/4λ ist.

6. Vorrichtung nach einem der Ansprüche 2 bis 5, wobei der Hauptwellenleiterabschnitt (27) eine Abmessung in der Richtung von L aufweist, die ein Vielfaches von im Wesentlichen 1/4λ ist.

7. Vorrichtung nach einem der vorhergehenden Ansprüche, ferner aufweisend einen Erweiterungsabschnitt (28), der an dem distalen Ende (22) gebildet ist, in dem die innere Elektrode (23) im Durchmesser erweitert ist und die äußere Elektrode (24) in der Dicke reduziert ist, um die Erweiterung aufzunehmen.

8. Vorrichtung nach Anspruch 7, wobei die Abmessung des Erweiterungsabschnitts (28) in der Richtung von L ein Vielfaches von im Wesentlichen 1/4λ ist.

9. Vorrichtung nach einem der vorhergehenden Ansprüche, wobei eine isolierende Beschichtung über dem distalen Ende (28) vorgesehen ist, um einen Kurzschluss zwischen dem inneren (23) und dem äußeren (24) Leiter zu verhindern.

10. Vorrichtung nach einem der vorhergehenden Ansprüche, wobei die elektrische Isolierung (25) zwischen der äußeren (24) und der inneren (23) Elektrode ein wärmeisolierendes Material umfasst.

11. Vorrichtung nach einem der vorhergehenden Ansprüche, wobei der Sensor ferner einen Wärmeisolierungsabschnitt (30) an seinem Verbindungsende (21) umfasst.

12. Vorrichtung nach einem der vorhergehenden Ansprüche, wobei die Verarbeitungselektronik eingerichtet ist, um eine Ausgabe auszulösen, die ausschließlich aus Amplituden einer Rückflussdämpfung abgeleitet wird.

13. Vorrichtung nach einem der Ansprüche 1 bis 11, wobei die Verarbeitungselektronik eingerichtet ist, um eine Ausgabe auszulösen, die aus einer Amplitude und Phasenänderungen einer Rückflussdämpfung abgeleitet wird.

14. Vorrichtung nach einem der vorhergehenden Ansprüche, wobei die Verarbeitungselektronik eingerichtet ist, um einen Spitzenanpassungsalgorithmus auf Spitzen der Rückflussdämpfung anzuwenden, um dann Änderungen einer Amplitude und/oder Bandbreite an diesen Spitzen einer Rückflussdämpfung zu bestimmen und dadurch einen Hinweis auf eine Änderung der Prozessvariablen zu liefern.

**Revendications**

1. Appareil conçu pour surveiller un changement dans une variable de processus dans un milieu en contact avec ledit appareil, ledit appareil incluant :

   un capteur à microondes comportant une extrémité de connexion (21) et une extrémité distale (22) destinée à être en contact avec le milieu ; et

   une électronique d'entraînement et de traitement connectée à l'extrémité de connexion (21) du capteur à microondes et configurée et conçue pour fournir des signaux d'entraînement à l'extrémité de connexion (21) dudit capteur, pour recevoir et traiter des signaux reçus résultant de la réflexion des signaux d'entraînement au niveau de l'extrémité distale (22) dudit capteur, et pour initier une sortie,

   dans lequel ledit capteur comprend un guide d'onde à microondes à extrémité ouverte formé par une électrode extérieure (24) et une électrode intérieure (23) maintenue coaxialement dans l'électrode extérieure (24) avec une isolation électrique (25) entre celles-ci, ledit capteur présentant une longueur L,

   dans lequel ledit capteur est configuré pour fonctionner dans une bande de fréquence f0 avec une longueur d'onde λ située substantiellement au centre de ladite bande, ledit capteur incluant au moins une désadaptation d'impédance présentant une dimension dans la direction de la longueur L associée à λ,

   **caractérisé en ce que**

   à ladite extrémité distale (22), ladite électrode intérieure (23) fait saillie au-delà de ladite électrode extérieure (24) dans la direction de L selon une quantité d'environ 1/10 λ ou moins, et

   dans lequel ladite électronique de traitement est configurée pour initier une sortie dérivée d'amplitudes de perte de rendement des signaux reçus par rapport aux signaux d'entraînement.

2. Appareil selon la revendication 1, dans lequel ledit capteur inclut une section de guide d'onde principal (27) d'impédance Z et dans lequel ladite désadaptation d'impédance est d'au moins 1,5 Z et de préférence substantiellement de 4 Z.

3. Appareil selon la revendication 2, dans lequel ladite désadaptation d'impédance est réalisée par une cavité résonante (26) située entre ladite section de guide d'onde principal (27) et ladite extrémité de connexion (21).

4. Appareil selon la revendication 2 ou la revendication 3, dans lequel ladite cavité résonante (26) est évacuée, ou remplie avec de l'air ou un matériau en mousse à faible permittivité.

5. Appareil selon l'une quelconque des revendications 1 à 4, dans lequel ladite désadaptation d'impédance présente une dimension dans la direction de la longueur L d'un multiple de substantiellement 1/4 λ.

6. Appareil selon l'une quelconque des revendications 2 à 5, dans lequel ladite section de guide d'onde principal (27) présente une dimension dans la direction de L correspondant à un multiple de substantiellement 1/4 λ.

7. Appareil selon l'une quelconque des revendications précédentes, incluant en outre une section d'élargissement (28) formée au niveau de ladite extrémité distale (22), dans laquelle ladite électrode intérieure (23) est élargie en diamètre et ladite électrode extérieure (24) est réduite en épaisseur pour s'adapter à l'élargissement.

8. Appareil selon la revendication 7, dans lequel la dimension de ladite section d'élargissement (28) dans la direction de L est un multiple de substantiellement 1/4 λ.

9. Appareil selon l'une quelconque des revendications précédentes, dans lequel un revêtement isolant est disposé sur ladite extrémité distale (28) pour empêcher un court-circuit entre lesdits conducteur intérieur (23) et extérieur (24).

10. Appareil selon l'une quelconque des revendications précédentes, dans lequel l'isolation électrique (25) entre lesdites électrodes extérieure (24) et intérieure (23) comprend un matériau d'isolation thermique.

11. Appareil selon l'une quelconque des revendications précédentes, dans lequel ledit capteur inclut en outre une section d'isolation thermique (30) au niveau de l'extrémité de connexion (21) de celui-ci.

12. Appareil selon l'une quelconque des revendications précédentes, dans lequel ladite électronique de traitement est configurée pour initier une sortie dérivée uniquement d'amplitudes de perte de rendement.

**13.** Appareil selon l'une quelconque des revendications 1 à 11, dans lequel ladite électronique de traitement est configurée pour initier une sortie dérivée de changements d'amplitude et de phase de perte de rendement.

**14.** Appareil selon l'une quelconque des revendications précédentes, dans lequel ladite électronique de traitement est configurée pour appliquer un algorithme d'ajustement de pic à des pics de perte de rendement, pour ensuite déterminer des changements d'amplitude et/ou de bande passante au niveau de ces pics de perte de rendement, et pour ainsi fournir une indication de changement dans la variable de processus.

Figure 1

(PRIOR ART)

Figure 2

| | Proposed sensor | | Standard coaxial sensor | |
|---|---|---|---|---|
| | Amplitude (dB) | Frequency (GHz) | Amplitude (dB) | Frequency (GHz) |
| $\varepsilon = 1$ | -1.87 | 4.8 | -0.65 | 6.3 |
| $\varepsilon = 3$ | -7.90 | 4.7 | -1.90 | 6.3 |
| $\varepsilon = 10$ | -9.24 | 4.5 | -5.30 | 6.3 |
| $\varepsilon = 80$ | -13.37 | 4.3 | -2.78 | 6.3 |

Figure 3

Figure 4

Resonant frequency change in Basmati rice

$y = -0.0001x^2 - 0.0022x + 5.1309$

Figure 5

Bandwidth change in Basmati rice

$y = 6.0812x + 219.15$

Figure 6

Figure 8

Figure 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5675259 A
- US 8629681 B
- US 2015276460 A1
- GB 2527794 A
- EP 1083414 A1